(19) Europäisches Patentamt
European Patent Office
Office européen des brevets

(11) Numéro de publication: **0 215 415 B1**

(12) **FASCICULE DE BREVET EUROPEEN**

(45) Date de publication de fascicule du brevet: **27.12.91**

(51) Int. Cl.$^5$: **C07C 45/28**

(21) Numéro de dépôt: **86112405.5**

(22) Date de dépôt: **08.09.86**

(54) **Procédé pour la fabrication de composés aldéhydiques ou cétoniques et aldéhydes et cétones obtenus par ce procédé.**

(30) Priorité: **09.09.85 IT 2209285**

(43) Date de publication de la demande: **25.03.87 Bulletin 87/13**

(45) Mention de la délivrance du brevet: **27.12.91 Bulletin 91/52**

(84) Etats contractants désignés: **AT BE CH DE FR GB LI NL SE**

(56) Documents cités:
**EP-A- 0 097 551**
**FR-A- 2 489 710**

**BULLETIN DE LA SOCIETE CHIMIQUE DE FRANCE, no. 5, 1969, pages 1481-1492, no. 267; H. MIMOUN et al.: "Nouveaux complexes peroxydiques covalents du molybdène et du tungstène avec les bases organiques"**

**TETRAHEDRON, vol. 26, 1970, pages 37-50, Pergamon Press, GB; H. MIMOUN et al.: "Epoxydation des oléfines par les complexes peroxydiques covalents du molybdène-VI"**

(73) Titulaire: **INTEROX Société Anonyme**
**Rue du Prince Albert, 33**
**B-1050 Bruxelles(BE)**

(72) Inventeur: **Campestrini, Sandro**
**Via Perini, 49**
**I-38100 Trente(IT)**
Inventeur: **di Furia, Fulvio**
**Via Manzoni, 60**
**I-35125 Padoue(IT)**
Inventeur: **Modena, Giorgio**
**Via S. Giovanni di Verdara, 75**
**I-35100 Padoue(IT)**
Inventeur: **Pasquato, Lucia**
**Via S. Marco, 1**
**I-35100 Padoue(IT)**

(74) Mandataire: **Lederer, Franz, Dr. et al**
**Lederer, Keller & Riederer Patentanwälte**
**Lucile-Grahn-Strasse 22**
**W-8000 München 80(DE)**

**EP 0 215 415 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé pour la fabrication de composés organiques de la famille des aldéhydes et des cétones. Elle concerne plus particulièrement la fabrication d'aldéhydes ou de cétones à partir d'oléfines par traitement de celles-ci au moyen d'un oxydant.

Il est connu de produire des méthylcétones par oxydation d'oléfines terminales au moyen d'oxygène moléculaire, en présence de complexes cationiques du rhodium III comme catalyseurs (JOURNAL OF MOLECULAR CATALYSIS, Vol. 7, janvier 1980, Lausanne, H. MIMOUN "The Role of Peroxymetallation in Selective Oxidative Processes" pages 1 à 29, * pages 12 et 13 *).

On a aussi proposé pour fabriquer des méthylcétones d'oxyder sélectivement les oléfines terminales au moyen de carboxylates-peroxydes de tert-butyle de palladium II (AMERICAN CHEMICAL SOCIETY, Vol. 102, No. 3, 30 janvier 1980, Washington D.C., H. MIMOUN, R. CHARPENTIER, A.MITSCHLER, J. FISCHER et R. WEISS "Palladium II tert-Butyl Peroxide Carboxylates. New Reagents for the Selective Oxidation of Terminal Olefins to Methyl Ketones. On the Role of Peroxymetalation in Selective Oxidative Processes" pages 1047 à 1054, * pages 1048 et 1049 *).

Ces procédés connus ne permettent cependant pas d'obtenir des aldéhydes ni des cétones autres que celles qui appartiennent à la classe des méthlycétones. Ils présentent de plus le désavantage de nécessiter un milieu anhydre sous peine d'inhiber la réaction ou de donner lieu à des précipitations de métal. Les complexes du rhodium nécessitent en outre la présence d'un alcool dans le milieu réactionnel.

Il est également connu de réaliser un clivage oxydant des doubles liaisons C=C des oléfines au moyen de tétroxyde de rhuténium pour obtenir des dérivés carbonylés (W.S. TRAHANOVSKY "Oxidation in Organic Chemistry" partie B, 1973, ACADEMIC PRESS, New-York, pages 177 à 227 : D.G. LEE et M. VAN DEN ENGH "Oxidation by Rhutenium Tetroxide" *pages 186 à 192*).

Ce procédé connu présente toutefois l'inconvénient de donner lieu à des sélectivités faibles : tous les types de dérivés carbonylès (aldéhydes, cétones et acides) sont produits ensemble et en mélange avec des produits de réactions concurrentes n'impliquant pas le clivage de la double liaison (diols).

On sait que des complexes peroxydiques covalents du molybdène et du tungstène avec un ligand du type diméthylformamide, diméthylacétamide, tétraméthylurée, hexaméthylphosphoramide (HMPT), octaméthylpyrophosphoramide, pyridine et 2,2'-bipyridine sont des agents d'oxydation sélectifs des oléfines en époxydes (BULLETIN DE LA SOCIETE CHIMIQUE DE FRANCE n° 5, 1969 pages 1481 à 1492 N° 267, H. Mimoun et al. "Nouveaux complexes peroxydiques covalents du molybdène et du tungstène avec les bases organiques" * pages 1481 à 1482 et 1491*).

Il est aussi connu d'employer des complexes de coordination d'un ligand, notamment l'hexaméthylphosphorotriamide et l'hexaéthylphosphorotriamide avec un composé peroxo du vanadium, du niobium ou du tantale pour fabriquer des composés aldéhydiques, cétoniques et époxydiques à partir d'oléfines (Demande de brevet européen EP-A-0097551 au nom de l'INSTITUT FRANCAIS DU PETROLE *revendications 1, 4, 7, exemples 9, 10 et 14 et description page 3, ligne 17 à page 4, ligne 26*).

L'invention remédie à ces inconvénients des procédés connus en fournissant un procédé nouveau de fabrication sélective de composés aldéhydiques ou cétoniques exempts d'acides et de diols qui n'exige pas nécessairement la présence d'un alcool, qui ne soit pas inhibé par la présence d'eau, même en quantités dépassant sa solubilité dans le milieu organique réactionnel, et qui permette l'obtention de cétones $R_1$-CO-$R_2$ où les groupes $R_1$ et $R_2$ peuvent tous les deux comporter plus d'un atome de carbone.

A cet effet, l'invention concerne un procédé pour la fabrication de composés organiques aldéhydiques ou cétoniques à partir d'oléfines selon lequel on réalise un clivage oxydant de la double liaison de l'oléfine au moyen d'un complexe de coordination d'un ligand et d'un composé peroxo d'un métal du groupe 6b en mettant en oeuvre des quantités réglées d'oléfine et de complexe pour que le rapport molaire de l'oléfine à l'oxygène actif du complexe soit compris entre 0,1 et 0,5.

Selon l'invention, par le terme composé organique aldéhydique, on entend désigner un composé organique carbonylé de formule générale

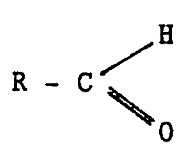

où le groupement R associé au radical aldéhyde représente un atome d'hydrogène ou une chaîne aliphatique linéaire ou ramifiée, substituée ou non par un ou plusieurs groupements fonctionnels (tels que,

par exemple, les groupements nitrile, halogène, alcool, amine, amide, éther et sulfonate), un groupement aromatique mono ou polycyclique substitué ou non par un ou plusieurs groupements fonctionnels (tels que ceux décrits ci-dessus), un groupement cyclanique mono ou polycyclique substitué ou non, un groupement de la classe des spiranes substitué ou non, ou une combinaison de deux ou plusieurs de ces groupements.

Par le terme composé organique cétonique, on entend désigner un composé organique carbonylé de formule générale $R_1$-CO-$R_2$ où $R_1$ et $R_2$ représentent les mêmes groupements que ceux déjà définis ci-dessus pour le groupement R de l'aldéhyde et pouvant tous les deux comporter plus d'un atome de carbone.

Selon l'invention, par oléfine on entend désigner des composés organiques comportant au moins une double liaison carbone-carbone tels que ceux qui suivent :

- les mono-oléfines aliphatiques comportant de 2 à 30 atomes de carbone et portant des substituants éventuels qui n'interfèrent pas avec les réactions d'époxydation comme par exemple les halogènes des halogénures d'allyle et les hydroxydes des alcools allyliques;
- les mono-oléfines cycloaliphatiques comportant de 4 à 20 atomes de carbone et portant des substituants éventuels qui n'interfèrent pas avec la réaction de clivage oxydant;
- les systèmes polycycliques contenant des doubles liaisons éthyléniques;
- les polyoléfines contenant des insaturations éthyléniques.

Des exemples de telles oléfines sont l'éthylène, le propylène, le butène-1, le butène-2, l'isobutène, le butadiène, les pentènes et notamment le pentène-1, le 2-méthyl-1-butène, le 3-méthyl-1-butène, le 2-méthyl-2-butène, le pypérilène, les hexènes -1, -2 et -3, les hexadiènes, le 2,3-diméthyl-2-butène, l'heptène-1, le 3-éthylpentène-2, l'octène-1, le diisobutylène, les 2,4,4-triméthylpentène-1 et -2, les octadiènes, le nonène-1, le décène-1, l'undécène-1, le dodécène-1, le tridécène-1, le tétradécèbe-1, le pentadécène-1, l'hexadécène-1, l'heptadécène-1, l'octadécène-1, le nonadécène-1, l'eicosène-1, les trimères et tétramères du propylène, les polybutadiènes, l'isopropène et les terpènes quels que les terpinènes, le limonène, le terpinolène, le sabinène, le pinène, le camphène, le myrcène, le cadinène, le cédrène, le santalène, le calarène, le colophène et les polyterpènes ainsi que leurs dérivés tels que le géraniol, le linalol et l'acétate de linalyle, le méthylènecylopropane, le cyclopentène et ses dérivés substitués par des groupes alkyles ou aryles, le cyclopentadiène, le cyclohexène et ses dérivés substitués par des groupes alkyles et aryles, le méthylcènecyclopentane, le cyclohexadiène, le méthylènecyclohexane, le norbornène, le cycloheptène, le vinylcylcohexane, le vinylcyclohexène, le styrène, le cyclooctène, les cylooctadiènes, le vinylnorbornène, l'indène substitué ou non, le tétrahydroindène, l'alphaméthylstyrène, et les alphaalkylstyrènes éventuellement substitués sur le noyau aromatique, le dicyclopentadiène, le divinylbenzène, les dihydronaphtèlènes substitués ou non, le cyclododécène, le cyclododécatriène, le stilbène, le 2,3-diphényl-2-butène, le diphénylbutadiène, les acides carboxyliques insaturés de tous types tels que l'acide acrylique, l'acide méthacrylique, les acides alpha- et béta-cyanoacryliques et leurs dérivés, l'acide crotonique, l'acide maléique et ses dérivés alkylés, l'acide vinylacétique et les acides gras insaturés parmi lesquels figurent plus particulièrement les acides oléique, linoléique, palmitoléique, linolénique, vaccinique, gadoléique, ricinoléique et éléostéarique et les graisses et huiles naturelles qui en contiennent ainsi que les esters de tous ces acides insaturés tels que les acrylates et méthacrylates d'alkyle, le maléate de diallyle, 7-hydroxy-5-heptanoate de méthyle, l'oléate de méthyle et les esters d'alcools insaturés tels que le carbonate d'allyle, le phtalate de diallyle, l'acétate d'allyle.

Selon l'invention, on réalise un clivage oxydant de la double liaison de l'oléfine au moyen d'un complexe de coordination d'un ligand et d'un composé peroxo d'un métal du groupe 6b du tableau périodique des éléments.

On entend désigner par clivage oxydant de la double liaison de l'oléfine, une rupture de la double liaison de l'oléfine et une oxydation concomitante de chacun de ses deux atomes de carbone pour former deux composés carbonylés selon le schéma réactionnel :

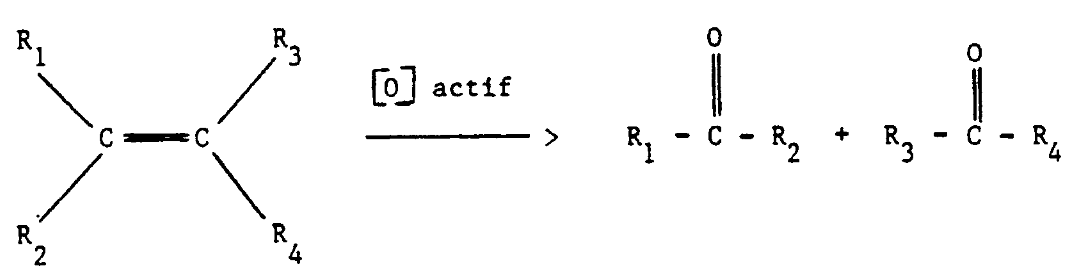

Dans ce schéma réactionnel :

$R_1$, $R_2$, $R_3$ et $R_4$ désignent chacun, un atome d'hydrogène ou un groupement carboné.

Le complexe de coordination d'un ligand et d'un composé peroxo d'un métal du groupe 6b est,selon l'invention, un composé de formule générale :

$M(O_2)_nL_mO_x$ où M représente le métal du groupe 6b;
L désigne le ligand;
O est un atome d'oxygène;
x est un nombre entier pouvant varier de 0 à 2;
m est un nombre entier pouvant varier de 1 à 6 et
n est un nombre entier pouvant varier de 1 à 3.

Des exemples de complexes de coordination d'un ligand et d'un composé peroxo d'un métal du groupe 6b utilisables dans le procédé selon l'invention sont les composés de formule :

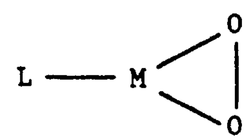

où M représente le métal du groupe 6b;
L désigne le ligand et
O est un atome d'oxygène

Des complexes préférés dans le cadre de l'invention, sont les complexes de coordination d'un ligand avec des composés oxo-diperoxo d'un métal du groupe 6b, à la valence 6, de formule :

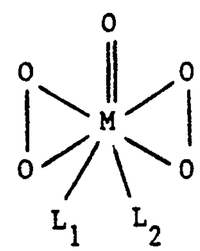

où M désigne le métal du groupe 6b;
$L_1$ désigne $H_2O$ ou un coordinat organique (ligand) et
$L_2$ désigne un coordinat organique (ligand)

Parmi ces complexes, ceux dans lesquels M désigne le molybdène ou le tungstène sont préférés.

Ces complexes sont préparés aisément par réaction en solution aqueuse d'anhydride ou d'acide de métal du groupe 6b avec du peroxyde d'hydrogène pour former le composé oxo-diperoxo, après quoi la solution contenant le composé est mise en contact avec le ligand pur ou en solution dans un solvant organique.

Selon l'invention, le ligand, ou coordinat employé peut avantageusement être sélectionné parmi les classes suivantes :

classe 1 : les amides tertiaires,
classe 2 : les phosphoramides,
classe 3 : les oxydes d'amines tertiaires aliphatiques et aromatiques,
classe 4 : les oxydes de phosphines et
classe 5 : les amines aromatiques.

Comme exemples de ligands appartenant à ces classes, qui conviennent dans le procédé selon l'invention, on peut citer, sans que la liste soit limitative :

classe 1 : le diméthylformamide, le diméthylacétamide et la tétraméthylurée;
classe 2 : l'hexaméthylphosphotriamide, l'hexaéthylphosphotriamide, l'hexabutylphosphotriamide, le tridodécylphosphotriamide, et l'octaméthylpyrophosphoramide;
classe 3 : l'oxyde de pyridine, l'oxyde de 4-picoline, l'oxyde de trioctylamine et l'oxyde de phénylpropylpyridine;
classe 4 : l'oxyde de triméthylphosphine;
classe 5 : la pyridine et la 2,2'-bipyridine.

Les proportions respectives de l'oléfine et de complexe peroxo qu'il convient de mettre en oeuvre dépendent d'un grand nombre de paramètres parmi lesquels figurent notamment la nature de l'oléfine et celle du ligand, la température et la pression maintenues pendant la réaction. Toutefois, il est nécessaire, pour assurer un rendement substantiel en aldéhyde ou en cétone, que le rapport molaire de l'oléfine à oxyder à l'oxygène actif apporté par le complexe peroxo ne dépasse pas la valeur 0,5. De bons résultats ont été obtenus lorsque ce rapport molaire était compris entre 0,1 et 0,5. Les meilleurs résultats ont été ceux où ce rapport molaire était compris entre 0,5 et 0,35.

Dans un mode d'exécution particulier du procédé selon l'invention, on effectue le clivage oxydant en phase homogène en solution dans un solvant organique. Dans ce mode d'exécution, on prépare au préalable le complexe peroxo par réaction en solution aqueuse entre le peroxyde d'hydrogène, les composés du métal du groupe 6b et le ligand en concentrations telles que le complexe peroxo formé précipite et puisse être isolé par filtration et essorage. Ce complexe est alors dissous dans le mélange réactionnel constitué d'une solution de l'oléfine à oxyder dans une solvant organique approprié. Par solvant approprié, on entend tout solvant capable de dissoudre à la fois l'oléfine et le complexe peroxo. Des exemples de tels solvants sont le benzène, le toluène, le xylène et leurs dérivés alkyl substitués; le dichlorméthane, le trichlorméthane, le tétrachlorméthane, le 1-chloréthane, le 1,1-dichloréthane, le 1,2-dichloréthane, le 1,1,2,2-tétrachloréthane, le 1-chlorpropane, le 2-chlorpropane, le 1,1-dichlorpropane, le 1,3-dichlorpropane, le 2,2-dichlorpropane, le 1,1,1-trichlorpropane, le 1,1,2-trichlorpropane, le 1,1,3-trichlorpropane, le 1,2,2-trichlorpropane, le 1,2,3-trichlorpropane, le tétrachlorpropane; le butane halogéné, le pentane halogéné, l'hexane halogéne, les hydrocarbures à nombre d'atomes de carbone supérieur à 6 substitués par un ou plusieurs atomes d'halogène; les hydrocarbures aromatiques chlorés tels que le chlorobenzène et les hydrocarbures aliphatiques linéaires ou ramifiés ayant de préférence 5 à 8 atomes de carbone.

Dans ce mode d'exécution du procédé selon l'invention, la durée et la température de la réaction sont variables et dépendent de divers facteurs, notamment de la nature de l'oléfine et de l'encombrement stérique de la structure de sa molécule, du type du complexe peroxo utilisé et particulièrement de la nature du ligand de coordination ainsi que de la pression à laquelle s'effectue la réaction. Elles sont à déterminer soigneusement dans chaque cas d'espèce par des essais de laboratoire. Pour des raisons économiques évidentes, on préfère généralement travailler à pression atmosphérique. On choisit le plus souvent une température inférieure au point d'ébullition du solvant organique sous la pression choisie. La durée peut être de quelques minutes à plusieurs heures.

Dans un autre mode d'exécution du procédé selon l'invention, qui est préféré, le clivage oxydant est exécuté au moyen de la technique connue de catalyse par transfert de phase. Par définition, cette technique fait usage de l'aptitude qu'ont certains catalyseurs (dans le cas présent, le complexe du métal du groupe 6b avec le ligand) de se transformer en dérivé actif dans une des deux phases, de passer ensuite dans l'autre phase où se trouve le substrat en solution et d'y promouvoir la réaction en se transformant à nouveau dans leur forme inactive après quoi, ils repassent dans la première phase où ils subissent à nouveau la tranformation en dérivé actif et ainsi de suite un nombre très élevé de cycles successifs ne dépendant que de la stabilité intrinsèque des deux formes active et inactive du catalyseur.

Dans la mise en oeuvre de ce mode d'exécution préféré du procédé selon l'invention, les deux phases que l'on met en présence sont, d'une part, une phase organique non miscible à l'eau, comprenant l'oléfine et, d'autre part, une phase aqueuse comprenant le complexe. Dans ce mode d'exécution du procédé selon l'invention, le complexe peut avantageusement être généré in situ dans la phase aqueuse, par réaction d'un dérivé inorganique d'un métal du groupe 6b, de peroxyde d'hydrogène et d'un ligand. Dès sa formation, le complexe peroxo passe de la phase aqueuse dans la phase organique où il vient oxyder l'oléfine en aldéhyde ou en cétone. Le complexe réduit par perte d'oxygène actif repasse ensuite en phase aqueuse pour y être réoxydé par le peroxyde d'hydrogène et recommencer ainsi un nouveau cycle. Ceux-ci peuvent se répéter très longtemps pourvu que la stabilité des formes oxydée et réduite du complexe soit suffisamment élevée. Dans ce mode d'exécution du procédé, il est souhaitable que la phase aqueuse contienne une petite quantité d'acide inorganique. La quantité optimum d'acide inorganique à mettre en oeuvre dépend de la nature du complexe et de sa solubilité dans l'eau, à l'état réduit. Elle est généralement adaptée pour maintenir le pH dans une gamme comprise entre 0,1 et 6,5.

Lorsque le ligand utilisé ne présente qu'une faible solubilité dans l'eau (inférieure à 200 mmoles/l), on peut avantageusement l'introduire dans la phase organique plutôt que dans la phase aqueuse. Dans ces conditions, la surface de contact importante entre les deux phases que l'on obtient moyennant une agitation

violente du milieu permet généralement la formation du complexe dans la phase aqueuse grâce à la dissolution limitée du ligand dans la phase aqueuse, due à son faible coefficient de partage entre la phase aqueuse et la phase organique.

Lorsque l'on met en oeuvre ce mode d'exécution préféré du procédé selon l'invention, le ligand doit être soigneusement choisi parmi les classes déjà citées plus haut et présenter en outre les propriétés simultanées suivantes :

- être capable de former avec le métal du groupe 6b un complexe peroxo qui présente une bonne solubilité dans la phase organique;
- être sans affinité pour le composé peroxo dans sa forme réduite, de façon à pemettre le retour du composé métallique et du ligand de la phase organique dans la phase aqueuse sitôt la réaction de réduction du complexe peroxo terminée.

Dans le mode d'exécution préféré par transfert de phase, la température et la durée de réaction peuvent varier dans de larges limites et sont fonctions des paramètres décrits plus haut auxquels il faut ajouter la vitesse plus ou moins grande à laquelle on peut réaliser le transfert de phase du complexe peroxo et de sa forme réduite. Elles peuvent être déterminées aisément par des essais de laboratoire. En général, la durée de la réaction peut varier de quelques minutes à plusieurs heures.

On préfère généralement travailler à pression atmosphérique à une température inférieure au point d'ébullition du solvant de la phase organique à pression normale.

Le peroxyde d'hydrogène peut être mis en oeuvre sous la forme d'une solution aqueuse. Avantageusement, on peut utiliser des solutions aqueuses peu concentrées. Des solutions contenant au moins 10 % en poids de préférence au moins 20 % en poids de peroxyde d'hydrogène conviennent bien. Pour des raisons économiques et de disponibilité, on ne met généralement pas en oeuvre de solutions contenant plus de 90 % en poids de peroxyde d'hydrogène et le plus souvent pas plus de 70 % en poids.

L'invention concerne aussi les aldéhydes ou les cétones obtenus au moyen du procédé selon l'invention tel qu'il a été décrit plus haut.

Les aldéhydes et les cétones selon l'invention sont des composés qui trouvent des applications dans l'industrie des solvants, des parfums et comme intermédiaires de synthèse de nombreux produits organiques.

Des particularités de l'invention ressortiront des exemples suivants qui décrivent des procédés de préparation d'aldéhydes et de cétones suivant l'invention.

Première série d'essais

Les exemples 1 et 2 concernent des essais dans lesquels le clivage oxydant est effectué en phase homogène, en milieu organique.

Exemple 1 (selon l'invention)

A une solution de 5,34 mmoles d'oxygène actif sous forme de complexe d'hexaméthylphosphotriamide de composé oxo-diperoxomolybdène ($MoO_5$-HMPT) dans 50 ml de 1,2-dichloréthane, on a ajouté 1,98 mmoles de styrène et on a ensuite maintenu le mélange à 40° C pendant 4 ½ heures, après quoi on a analysé les produits de la réaction par chromatographie gazeuse. On a trouvé 0,35 mmole de benzaldéhyde et 0,08 mmole d'alpha-tolualdéhyde. Le taux de conversion par rapport à l'oléfine consommée à été de 43 %. Les résultats sont reproduits au tableau 1.

Exemple 2 (selon l'invention) :

On a reproduit l'exemple 1 avec de l'alpha-méthylstyrène. La durée de la réaction a été de 5 heures. Les résultats des analyses chromatographiques sont donnés au tableau 2.

T A B L E A U 1

| Oléfine | | O actif, mmoles | taux de conversion de l'oléfine consommée, % | Produits | |
|---|---|---|---|---|---|
| type | quantité, mmoles | | | type | quantité, mmoles |
| 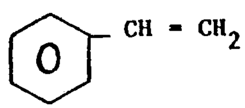 | 1,98 | 5,34 | 43 | 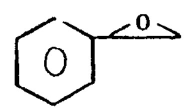 | 0,08 |
| | | | | 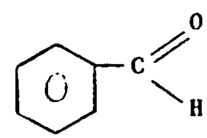 | 0,35 |
| | | | | 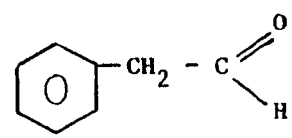 | 0,08 |

TABLEAU 2

| Oléfine type | Oléfine quantité, mmoles | O actif, mmoles | taux de conversion de l'oléfine consommée, % | Produits type | Produits quantité, mmoles |
|---|---|---|---|---|---|
| 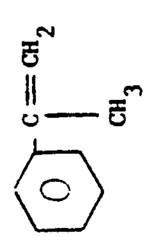 | 1,30 | 2,62 | 90 | 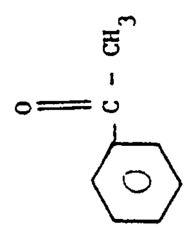 | 0,19 |
| | | | | 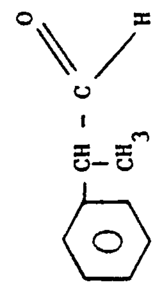 | 0,63 |

Seconde série d'essais

Les exemples 3 (de référence) et 4 et 5, (conformes à l'invention) concernent des essais mettant en oeuvre la technique de catalyse par transfert de phase.

Exemple 3 (de référence)

A une solution de 100 mmoles de trans-2-butène et 1 mmole d'hexabutylphosphotriamide (HBPT) dans 25 ml de 1,2-dichloréthane, on a ajouté 2 ml d'une solution aqueuse contenant 0,5 mmole de $Na_2MoO_4$, 20,5 mmoles de $H_2O_2$ et une quantité réglée d'acide sulfurique pour amener son pH à 2.

Le mélange a ensuite été soumis à forte agitation et chauffé à 50° C. La température et l'agitation ont été maintenues pendant 20 heures.

Après refroidissement et une légère alcalinisation avec NaOH 0,1N, on a séparé les deux phases.

La phase aqueuse a été analysée par iodométrie et a révélé une consommation en peroxyde de 98 % et un contenu en molybdate pratiquement égal à la quantité initialement introduite. Cette phase aqueuse a pu être recyclée telle quelle après avoir été réadditionnée de l'$H_2O_2$ nécessaire et réajustée à pH 2.

La phase organique, analysée par chromatographie gazeuse (méthode du standard interne) a indiqué la formation de 12,5 mmoles d'époxyde de trans-2-butène.

Exemple 4 (selon l'invention)

A une solution de 10 mmoles de trans-beta-méthylstyrène et 1 mmole de tridodécylphosphotriamide (TDPT) dans 25 ml de 1,2 dichloréthane, on a ajouté 2 ml d'une solution aqueuse contenant 0,5 mmole de $Na_2MoO_4$, 30 mmoles de $H_2O_2$ et une quantité réglée d'acide sulfurique pour amener son pH à 1,1.

Le mélange a ensuite été soumis à forte agitation et chauffé à 50° C. La température et l'agitation ont été maintenues pendant 24 heures.

Après refroidissement et une légère alcalinisation avec NaOH 0,1N, on a séparé les deux phases.

La phase aqueuse a été analysée par iodométrie et a révélé une consommation en peroxyde de 79 %, un contenu en molybdate pratiquement égal à la quantité initialement introduite et une quantité d'oléfine non consommée inférieure à 1 %. Cette phase aqueuse a pu être recyclée telle quelle après avoir été réadditionnée de l'$H_2O_2$ nécessaire et réajustée à pH 1,1.

La phase organique, analysée par chromatographie gazeuse (méthode du standard interne) a indiqué un rendement en benzaldéhyde, calculé par rapport à l'oléfine consommée. de 92 % en poids. Le benzaldéhyde a été séparé de la phase organique par distillation fractionnée sous vide. Les produits qui ont distillé en tête sont le solvant et l'oléfine en excès n'ayant pas été oxydée et qui peuvent être recyclés. Le résidu lourd de distallation a été constitué principalement de TDPT qui peut être réutilisé, moyennant purification éventuelle appropriée.

Exemple 5 (selon l'invention)

On a reproduit l'exemple 4 en mettant en oeuvre 10 mmoles de trans-beta-méthylstyrène et 20 mmoles de $H_2O_2$, les quantités de solvant et de $Na_2MoO_4$ restant inchangées.

La durée de la réaction a été aussi de 24 heures à 50° C.

Le taux de conversion de l'eau oxygénée a été de 100 % et celui de l'oléfine de 70 %.

Dans le phase organique on a dosé du benzaldéhyde (rendement : 69 % par rapport à l'oléfine consommée) et de l'oxyde de styrène (rendement : 14 % par rapport à l'oléfine consommée).

## Revendications

**1.** Procédé pour la fabrication de composés organiques aldéhydiques ou cétoniques à partir d'oléfines, caractérisé en ce qu'on réalise un clivage oxydant de la double liaison de l'oléfine au moyen d'un complexe de coordination d'un ligand et d'un composé peroxo d'un métal du groupe 6b, le rapport molaire de l'oléfine à l'oxygène actif du complexe étant compris entre 0,1 et 0,5.

**2.** Procédé selon la revendication 1, caractérisé en ce que le métal est choisi parmi le molybdène et le tungstène.

**3.** Procédé selon la revendication 1 ou 2, caractérisé en ce que le ligand est choisi parmi les amides tertiaires, les phosphoramides, les oxydes d'amines aliphatiques et aromatiques, les oxydes de phosphines et les amines aromatiques.

**4.** Procédé selon la revendication 3, carctérisé en ce qu le phosphoramide est choisi parmi l'hexaéthylphosphoramide, l'hexaéthylphosphotriamide, l'hexabutylphosphotriamide, le tridodécylphosphotriamide et l'octométhylpyrophosphoramide.

5. Procédé selon la revendication 3, caractérisé en ce que l'oxyde d'amine est sélectionné parmi l'oxyde de trioctylamine et l'oxyde de phénylpyridine.

6. Procédé selon l'une quelconque des revendications 1 à 5, caractérisé en ce que le composé peroxo est un composé oxo-diperoxo.

7. Procédé selon l'une quelconque des revendication 1 à 6, caractérisé en ce qu'on exécute le cliavage oxydant en phase homogène dans un solvant organique.

8. Procédé selon l'une quelconque des revendications 1 à 6, caractérisé en ce que le clivage oxydant est exécuté par la technique de catalyse par transfert de phase, dans laquelle on met en oeuvre, d'une part, une phase organique non miscible à l'eau, comprenant l'oléfine et, d'autre part, une phase aqueuse comprenant le complexe.

9. Procédé selon la revendication 8, caractérisé en ce qu'on génère le complexe in situ dans la phase aqueuse par dissolution dans ladite phase aqueuse d'un composé hydrosoluble du métal, d'un ligand et de peroxyde d'hydrogène.

10. Procédé selon la revendication 9, caractérisé en ce que l'introduction du ligand dans le milieu réactionnel se fait dans la phase organique.

11. Procédé selon la revendication 9 ou 10, caractérisé en ce que le peroxyde d'hydrogène est mis en oeuvre à l'état d'une solution aqueuse contenant entre 10 et 90 % en poids de peroxyde d'hydrogène.

12. Procédé selon l'une quelconque des revendications 9 à 11, caractérisé en ce que le rapport molaire de l'oléfine au peroxyde d'hydrogène est compris entre 0,1 et 0,5.

13. Procédé selon l'une quelconque des revendications 1 à 12, caractérisé en ce qu'on effectue le clivage oxydant à une température inférieure au point d'ébullition du solvant organique à la pression régnant dans le milieu réactionnel.

## Claims

1. Process for the production of organic aldehyde or ketone compounds on the basis of olefins characterized in that an oxidising cleavage of the double bond of the olefin by means of a coordination complex of a ligand and a peroxo compound of a metall belonging to group 6b is realised, wherein the molar ratio of the olefin to the active oxygene of the complex is within 0,1 and 0,5.

2. Process according to claim 1, characterized in that the metal is selected among molbydenum and tungsten.

3. Process according to claim 1 or 2, characterized in that the ligand is selected among tertiary amides, phosphorus amides, aliphatic and aromatic amine oxides, phosphine oxides and aromatic amines.

4. Process according to claim 3, characterized in that the phosphorus amide is selected among hexaethylphosphoramide, hexaethylphosphotriamide, hexabutylphosphotriamide, tridodecylphosphotriamide and octomethylpyrophosphoramide.

5. Process according to claim 3, characterized in that the amine oxide is selected among trioctylamine oxide and phenylpyridine oxide.

6. Process according to one of claims 1 to 5, characterized in that the peroxo compound is a oxo-diperoxo compound.

7. Process according to one of claims 1 to 6, characterized in that the oxidising cleavage is carried out in a homogeneous phase in an organic solvent.

8. Process according to one of claims 1 to 6 characterized in that the oxidising cleavage is carried out by

the catalysis technique by means of phase transfer in which on the one hand an organic phase non-miscible with water comprising the olefin and on the other hand an aqueous phase comprising the complex is used.

9. Process according to claim 8, characterized in that the complex is generated in situ in the aqueous phase by dissolution in the aqueous phase of a hydrosoluble compound of the metal, a ligand and the hydrogen peroxide.

10. Process according to claim 9 characterized in that the ligand is introduced into the reaction medium in the organic phase.

11. Process according to claim 9 or 10 characterized in that the hydrogen peroxide is used in the state of an aqueous solution containing between 10 and 90% by weight of hydrogen peroxide.

12. Process according to one of claims 9 to 11 characterized in that the molar ratio of the olefin to the hydrogen peroxide is between 0,1 and 0,5.

13. Process according to one of claims 1 to 12, characterized in that the oxidising cleavage is carried out at a temperature lower than the boiling point of the organic solvent at the pressure present in the reaction medium.

**Patentansprüche**

1. Verfahren zur Herstellung von organischen Aldehyd- oder Ketonverbindungen auf der Basis von Olefinen, dadurch gekennzeichnet, daß man eine oxidierende Spaltung der Doppelbindung des Olefins mittels eines Koordinationskomplexes eines Liganden und einer Peroxoverbindung eines Metalls der Gruppe 6b durchführt, wobei das molare Verhältnis des Olefins zu dem aktiven Sauerstoff des Komplexes zwischen 0,1 und 0,5 liegt.

2. Verfahren nach Anspruch 1, dadurch gekennzeichnet, daß das Metall ausgewählt ist unter Molybdän und Wolfram.

3. Verfahren nach Anspruch 1 oder 2, dadurch gekennzeichnet, daß der Ligand ausgewählt ist unter den tertiären Amiden, den Phosphoramiden, den aliphatischen und aromatischen Aminoxyden, den Phosphinoxyden und den aromatischen Aminen.

4. Verfahren nach Anspruch 3, dadurch gekennzeichnet, daß das Phosphoramid ausgewählt ist unter Hexaethylphosphoramid, Hexaethylphosphotriamid, Hexabutylphosphotriamid, Tridodecylphosphotriamid und Octomethylpyrophosphoramid.

5. Verfahren nach Anspruch 3, dadurch gekennzeichnet, daß das Aminoxyd ausgewählt ist unter Trioctylaminoxyd und Phenylpyridinoxyd.

6. Verfahren nach einem der Ansprüche 1 bis 5, dadurch gekennzeichnet, daß die Peroxoverbindung eine Oxo-Diperoxo-Verbindung ist.

7. Verfahren nach einem der Ansprüche 1 bis 6, dadurch gekennzeichnet, daß man die oxidierende Spaltung in homogener Phase in einem organischen Lösungsmittel durchführt.

8. Verfahren nach einem der Ansprüche 1 bis 6, dadurch gekennzeichnet, daß die oxidierende Spaltung durch die Katalysetechnik mittels Phasentransfer durchgeführt wird, wobei man einerseits eine mit Wasser nichtmischbare organische Phase, die das Olefin umfaßt, und andererseits eines wässrige Phase, die den Komplex umfaßt, einsetzt.

9. Verfahren nach Anspruch 8, dadurch gekennzeichnet, daß man den Komplex in situ in der wässrigen Phase durch Auflösen in dieser wässrigen Phase einer wasserlöslichen Verbindung des Metalls, des Liganden und des Wasserstoffperoxyds erzeugt.

10. Verfahren nach Anspruch 9, dadurch gekennzeichnet, daß die Einführung des Liganden in das Reaktionsmilieu in der organischen Phase geschieht.

11. Verfahren nach Anspruch 9 oder 10, dadurch gekennzeichnet, daR das Wasserstoffperoxyd im Zustand einer wässrigen Lösung, die zwischen 10 und 90 Gew.-% Wasserstoffperoxyd enthält, eingesetzt wird.

12. Verfahren nach einem der Ansprüche 9 bis 11, dadurch gekennzeichnet, daß das molare Verhältnis des Olefins zu Wasserstoffperoxyd zwischen 0,1 und 0,5 liegt.

13. Verfahren nach einem der Ansprüche 1 bis 12, dadurch gekennzeichnet, daß die oxidierende Spaltung bei einer Temperatur, die niedriger ist als der Siedepunkt des organischen Lösungsmittels unter dem in dem Reaktionsmilieu herrschenden Druck durchgeführt wird.